Europäisches Patentamt

European Patent Office

Office européen des brevets

(19)

(11) Numéro de publication: **0 417 002 A1**

# DEMANDE DE BREVET EUROPEEN

(21) Numéro de dépôt: **90402447.8**

(22) Date de dépôt: **06.09.90**

(51) Int. Cl.5: **A61K 37/18**, A61K 35/14, C12Q 1/02

(30) Priorité: **08.09.89 FR 8911744**

(43) Date de publication de la demande:
**13.03.91 Bulletin 91/11**

(84) Etats contractants désignés:
**BE CH DE ES FR GB IT LI**

(71) Demandeur: **IMEDEX SOCIETE ANONYME**
**58 avenue Leclerc**
**F-69007 Lyon(FR)**

(72) Inventeur: **Tiollier, Jérôme**
**41 Quai Ja r**
**F-69 009 Lyon(FR)**
Inventeur: **Uhlrich, Sylvie**
**Immeuble "Les Acacias", 282 Chemin de**
**Fontanière**
**F-69 350 La Mulatiere(FR)**
Inventeur: **Tayot, Jean-Louis**
**1 rue des Greffières**
**F-69 890 La Tour de Salvagny(FR)**

(74) Mandataire: **Bernasconi, Jean et al**
**CABINET LEMOINE ET BERNASCONI 13,**
**Boulevard des Batignolles**
**F-75008 Paris(FR)**

(54) **Application d'hydrolysats de globines pour la stimulation cellulaire, procédé de test de l'activité de ces substances, produits pharmaceutiques et cosmétiques obtenus et procédé de préparation d'hydrolysats de globines.**

(57) Des hydrolysats de globines, notamment extraits de cruor, sont utilisés pour la préparation de produits pharmaceutiques, notamment pour la cicatrisation, et de produits cosmétiques d'activation du métabolisme et de la multiplication cellulaires pour les soins des cheveux et/ou de la peau.

Un test de multiplication cellulaire comprenant l'incubation de ces hydrolysats avec des cellules animales dans un milieu dépourvu de sérum animal et en l'absence de facteurs de croissance dans l'hydrolysat, permet d'en tester l'activité.

Les conditions de chauffage de l'hydrolysat permet l'obtention d'un hydrolysat répondant positivement ou négativement audit test.

L'invention est relative à l'application d'hydrolysats de globines, tels que des extraits de cruor, pour la stimulation de l'activité métabolique et de la croissance ou multiplication de divers types cellulaires. Elle a trait à des produits pharmaceutiques ainsi qu'à des produits de cosmétologie incorporant ces extraits ou hydrolysats ainsi qu'à un procédé permettant de tester l'efficacité de ces produits.

En cosmétique, y compris dans le domaine des produits cosmétiques pour les soins de la peau, on utilise des préparations contenant des protéines ou des produits plus ou moins bien définis d'origine protéique.

On a d'ailleurs déjà proposé (brevet FR-A-2 609 393) d'une façon générale d'utiliser comme produits cosmétiques toutes sortes de substances azotées, issues par exemple de plasma, de sérum sanguin, de placenta, etc., par exemple par hydrolyse, notamment par hydrolyse enzymatique.

Par ailleurs, on connaît déjà un certain nombre de procédés pour tester l'efficacité de ces substances.

Dans le brevet EP-A-O 120 722, on compare des hydrolysats d'élastine ou de collagène encapsulés dans une phase lipidique aux mêmes hydrolysats non encapsulés, dans un test de multiplication cellulaire en milieu nutritif classique contenant du sérum de veau et donc des facteurs usuels de croissance ou de multiplication cellulaire. Ce test cherche à déterminer si l'encapsulation présente un avantage pour la multiplication cellulaire dans les domaines de la cosmétologie et de la pharmacie. Il ne permet cependant pas de déterminer un niveau d'efficacité d'une substance candidate à une application en cosmétologie qui ne serait associée ni avec des liposomes ni avec du sérum animal.

M.C. MARTINI et al., dans International Journal of Cosmetic Science 3, 69-82 (1981), décrivent un test de l'activité de substances à usage cosmétique par oxygraphie, c'est-à-dire par la mesure de l'augmentation de la consommation cellulaire d'oxygène. Or, une telle augmentation ne signifie pas toujours un accroissement de la multiplication cellulaire.

La demande de brevet européen EP-A-O 237 998 décrit un test permettant de mesurer l'activité multiplicatrice cellulaire d'un peptide, comprenant la mise en présence de la substance à tester avec des cellules animales contenues dans un milieu de culture, l'incubation et le contrôle de la multiplication cellulaire desdites cellules par rapport à un témoin ne recevant pas la substance à tester. Le test décrit s'applique à des polypeptides bien définis, n'incluant pas des globines, et ne prévoit pas l'absence de facteurs de croissance.

Par ailleurs, le brevet français n° 2 628 118 décrit des fractions peptidiques obtenues par hydrolyse enzymatique d'hémolysats de cruor. Il prévoit l'application de ces fractions peptidiques comme constituants de milieux de culture biologique ou cellulaire.

Un premier objectif de l'invention est de fournir des produits à la fois efficaces pour la réparation tissulaire et pour la multiplication cellulaire, sans être dangereux et notamment ne requérant pas une surveillance médicale.

Un autre objectif est de fournir un nouveau procédé pour tester l'efficacité de ces produits.

L'invention a pour objet l'application

L'invention a pour objet l'application d'hydrolysats de globines, tels que, notamment, des extraits de cruor, à la préparation de produits pharmaceutiques, notamment pour la cicatrisation, et à la préparation de produits cosmétiques d'activation du métabolisme et de la multiplication cellulaires pour les soins des cheveux et/ou de la peau.

De préférence, on utilise les hydrolysats répondant positivement à un test dans lequel on met la substance ou préparation de globine hydrolysée ou provenant de l'hydrolysat, de préférence à différentes concentrations, en présence de cellules animales contenues dans un milieu de culture dépourvu de sérum animal, par exemple sérum de veau foetal (SVF), on laisse incuber, puis on contrôle l'existence et, éventuellement, l'ampleur d'une multiplication desdites cellules par rapport à un milieu témoin ne recevant pas la substance ou la préparation testée.

L'invention a également pour objet un procédé de test de l'activité d'hydrolysats de globine, tels que, notamment, de cruor, caractérisé en ce que l'on met l'hydrolysat, de préférence à différentes concentrations, en présence de cellules animales contenues dans un milieu de culture dépourvu de sérum de veau foetal (SVF), on laisse incuber, puis on contrôle l'existence et, éventuellement, l'ampleur d'une multiplication desdites cellules par rapport à un milieu témoin ne recevant pas l'hydrolysat testé.

Si la préparation à tester contient des facteurs de croissance cellulaire, ceux-ci peuvent être préalablement éliminés, par exemple par hydrolyse enzymatique complémentaire puis chauffage, par exemple hydrolyse par la papaïne, la pepsine ou d'autres protéases, puis chauffage à 120 °C pendant 30 à 40 mn.

On détermine ainsi les hydrolysats qui sont efficaces ainsi que leurs plages de concentration utiles.

Au test selon l'invention, on peut éventuellement associer un test de stimulation de l'incorporation de thymidine tritiée dans l'ADN de cellules animales.

Le test de stimulation de la prolifération de cellules animales est effectué de préférence sur des fibroblastes.

Le milieu de culture des fibroblastes comprend de préférence un mélange DMEM/HAM F12 additionné, de préférence, de substances telles que insuline, transferrine, sélénite, albumine ou autres protéines, fibronectine, antibiotiques. Le milieu préféré est additionné de 5 ug/ml d'insuline, de 5 $\mu$g/ml de transferrine, de 3.10$^{-8}$ M de sélénite, de 2 mg/ml d'albumine, de 2 $\mu$g/ml de fibronectine et de 50 $\mu$g/ml de gentamycine.

De préférence, on incube la culture avec l'hydrolysat à raison de 50 000 cellules environ par ml de milieu de préférence pour une surface de 2 cm², pendant environ 5 jours à 37° C, en ambiance humide et en présence de 5 % environ de $CO_2$, puis on effectue un comptage des cellules.

De préférence, on change le milieu de culture au deuxième jour de l'incubation.

Les fibroblastes utilisés peuvent être des fibroblastes diploïdes de hamster.

Le test de stimulation de l'incorporation de thymidine tritiée est effectué de préférence sur des fibroblastes et notamment sur des fibroblastes cutanés humains.

De préférence, les fibroblastes sont ensemencés dans un milieu de culture comprenant du HAM F10 additionné de 150 U/ml de pénicilline, de 150 U/ml de streptomycine, de 100 $\mu$g/ml de fongizone et de 12 % de SVF.

De préférence, au jour 0, on ensemence environ 50 000 cellules par ml de milieu de culture pour une surface de préférence de l'ordre de 2 cm² ; au jour 7, on remplace le milieu par du HAM F10 additionné de 1 % de SVF ; au jour 9, on ajoute l'hydrolysat à tester ainsi que 1 $\mu$Ci/ml de thymidine tritiée ; au jour 10, on effectue un lavage puis on mesure la radioactivité.

La déposante a appliqué le test selon l'invention à des hydrolysats de globine tels que des extraits de cruor, de préférence décolorés, notamment de cruor d'origine bovine.

L'invention a également pour objet des produits cosmétiques d'activation du métabolisme et de la multiplication cellulaires pour les soins des cheveux et/ou de la peau, ou des produits pharmaceutiques de dermatologie, caractérisés en ce qu'ils comportent une ou plusieurs substances provenant de l'hydrolysat de globine ou constitués par lui, ou des produits en dérivant. De préférence, ces substances sont capables de répondre positivement au procédé de test selon l'invention.

De préférence, ces substances sont des extraits de cruor. Elles peuvent avantageusement être associées à des agents ou véhicules cosmétiques usuels. Dans un mode de mise en oeuvre particulièrement perfectionné de l'invention, elles ont été épurées de facteurs de croissance cellulaires de type cytokines tels que FGF, PDGF, EGF, etc. si ceux-ci étaient présents au départ.

Les produits cosmétiques selon l'invention sont remarquables par leur activation contrôlée du métabolisme et de la multiplication cellulaires, et sont d'une efficacité remarquable mais ne demandant pas un environnement médical.

L'invention a encore pour objet un procédé de préparation d'hydrolysats de globine tels que, notamment, d'extraits de cruor, dans lequel on chauffe la préparation d'hydrolysat de façon que celle-ci réponde positivement ou négativement au procédé de test décrit ci-dessus.

L'invention va maintenant être décrite à l'aide d'exemples de réalisation montrant la mise en oeuvre des susdits tests appliqués à des hydrolysats selon l'invention.


Exemple 1 : hydrolysat de globine bovine.


1) Préparation de l'hydrolysat de globine bovine.

Cette préparation peut être effectuée de différentes manières :
a) 100 l de cruor, contenant 270 g d'hémoglobine par litre, sont dilués par 300 l d'eau permutée, de manière à obtenir une solution à 67,5 g/l dont le pH est de 7,2.

400 g de papaïne T400 purifiée, en solution dans 2 litres d'eau, sont ajoutés et le mélange est homogénéisé par agitation.

La température est alors portée à 72° C et maintenue à cette valeur pendant 1 h. Passé ce temps, le pH s'est stabilisé à 6,4 et 18 l d'$H_2SO_4$ 1N sont introduits pour l'ajuster à 5. L'incubation est poursuivie pendant encore 30 mn à environ 70° C.

On effectue une clarification du mélange par filtration sur filtre rotatif. A l'issue de cette étape, 430 l de solution décolorée sont obtenus. Après autoclavage 40 mn à 120° C, la concentration en matières protéiques est de 40 g/l et l'on obtient un hydrolysat I actif dans le test selon l'invention.

a') On répète a) avec un autoclavage de 1 h à 132° C et l'on obtient un hydrolysat II inactif dans le test selon l'invention, mais actif dans les tests usuels, y compris dans le test de consommation d'oxygène.

a") On répète a) avec une autoclavage de 3h30 à 120°C (hydrolysat III). Les résultats sont sensiblement identiques à ceux de a').

b) Le produit de départ est du cruor de sang bovin préparé à l'abattoir. Sa concentration en hémoglobine est de 260 g/l. 25 ml de cruor sont ajoutés à 125 ml d'eau déminéralisée pour provoquer l'hémolyse des hématies et l'obtention d'une solution à 43,3 g d'hémoglobine par litre.

La solution est amenée à 37°C et le pH ajusté à 3 par quelques gouttes d'acide sulfurique concentré.

25 mg de pepsine T1000 selon CODEX 49 en solution dans 1 ml d'$H_2SO_4$ 0,1N sont ajoutés et la solution est maintenue à 37°C avec agitation pendant 15 minutes.

Le mélange est ensuite rapidement porté à 80°C et maintenu à cette température en agitant pendant 20 minutes.

10 minutes de centrifugation à 9000 tr/mn permettent de séparer un surnageant de 96 ml qui est neutralisé à pH 7 par NaOH. Après 2 heures de repos à température ambiante, une nouvelle centrifugation de 10 minutes permet d'écarter un léger précipité marron. Le nouveau surnageant obtenu est jaune pâle, parfaitement limpide et possède une concentration en protéines de 44 g/l pour un volume de 95 ml. Le rendement en protéines est donc de 80%.

c) 300 ml de cruor bovin dilué 6 fois, contenant 14 g d'hémoglobine, sont portés à 37°C et le pH est ajusté à 2 par de l'acide sulfurique concentré.

40 mg de pepsine T1000, en solution dans 1 ml d'$H_2SO_4$ 0,1N , sont immédiatement introduits et la réaction se déroule pendant 30 minutes. Au bout de ce laps de temps, le pH s'est stabilisé à 2,6.

La solution est alors portée à 80°C pendant 30 minutes puis centrifugée 15 minutes à 9000 tr/mn.

Les 228 ml de surnageant jaune pâle limpide récupérés contiennent 11,6 g de protéines, ce qui correspond à un rendement de 83 %.

d) Du cruor bovin est dilué de manière à obtenir une solution à 50 g d'hémoglobine par litre.

150 ml de cette solution sont traités à pH 2 et 37°C pendant 30 minutes par 10 mg de pepsine T1000, puis chauffés à 70°C pendant encore 30 minutes avant d'être centrifugés 10 minutes à 9000 tr/mn.

Le surnageant clair et limpide obtenu est neutralisé à pH 7 et laissé quelque temps à température ambiante. Le léger précipité formé est éliminé par centrifugation. Les 116 ml de produit obtenus contiennent 6,4 g de protéines.

Par diafiltration puis ultrafiltration, la solution est dessalée et concentrée, puis séchée par lyophilisation.

La poudre blanche obtenue est inodore et insipide. Sa masse est de 6,2 g et son analyse permet de tirer les conclusions suivantes :

| - humidité | 3,2 % |
| - matières minérales résiduelles | 1,5 % |
| - teneur pondérale en peptides | 5,9 g |

L'électrophorèse sur gel de polyacrylamide fait apparaître une bande homogène de masse moléculaire apparente de 3500 à 4000 Daltons.

e) 50 l de cruor bovin frais correspondant à 14 kg d'hémoglobine sont mélangés à 250 l d'eau permutée. 35 g de pepsine T1000 en solution dans 3 l d'$H_2SO_4$ 0,1N sont ensuite ajoutées au mélange et le pH est ajusté à 2,1 par 27,5 l d'$H_2SO_4$ 1N.

Le mélange est alors porté à 37°C et maintenu à cette température pendant 30 minutes avec agitation. En fin de réaction, le pH s'est stabilisé à 2,4.

Le mélange est enfin amené à 75°C et maintenu ainsi pendant encore une demi-heure.

Après refroidissement à 44°C, le produit est filtré sur un filtre à plaques et 180 l de filtrat sont récupérés. Le rétentat est lavé sur le filtre par 125 l d'une solution de NaCl à 1,5 g/l, pH 2,5 par $H_2SO_4$ et 150 l de solution de lavage sont récupérés et mélangés au premier filtrat.

Les 330 l de solution ainsi obtenus sont neutralisés à pH 7,2 par 11,15 l de NaOH 2N.

Après une nuit à +4°C, le mélange est centrifugé et le culot est éliminé. Le surnageant est dessalé par diafiltration, puis concentré par ultrafiltration.

Le concentrat est alors séché par atomisation et 9 kg de poudre pratiquement blanche de dérivés de globine sont récoltés.

Cette poudre peut aisément être remise en solution dans de l'eau, quel que soit le pH de départ.

2) Stimulation de la prolifération de fibroblastes CCL39

La lignée fibroblastique CCL39 (fibroblastes diploïdes de hamster - souche ATCC) est cultivée dans un milieu défini, constitué d'un mélange DMEM/HAM F12 additionné de 5 μg/ml d'insuline, de 5 μg/ml de transferrine, de $3.10^{-8}$ M de sélénite, de 2 mg/ml d'albumine, de 2 μg/ml de fibronectine et de 50 μg/ml de gentamycine.

Les tests de prolifération cellulaire sont réalisés sur des plaques 24 puits (FALCON réf.3047) avec, par puits, au jour 0(J0), 50 000 cellules dans 1 ml de milieu contenant l'hydrolysat I à la concentration désirée pour une surface de l'ordre de 2 cm². La plaque est mise en incubation pendant 5 jours à 37°C, en chambre humide, en présence de 5 % de $CO_2$. Le milieu (contenant l'hydrolysat de globine) est renouvelé à J2. Le comptage est effectué après trypsinisation des cellules.

On peut également ne pas changer le milieu à J2, ce qui entraîne une diminution de l'effet final observé.

Dans les mêmes conditions, on a testé un extrait placentaire actuellement commercialisé en France et incorporé dans diverses spécialités cosmétiques.

Les résultats obtenus avec des cellules CCL39 au 50e passage sont rassemblés dans le tableau I.

A des concentrations protéiques finales supérieures à 1 mg/ml, l'hydrolysat de globine, préparé selon l'exemple 1, induit de façon significative la stimulation de la prolifération des fibroblastes d'un facteur variant de 45% à 427% par rapport au milieu témoin. Dans les mêmes conditions, l'extrait placentaire n'induit aucune stimulation significative.

3) Stimulation de la consommation d'oxygène d'un broyat de foie de souris (selon le procédé décrit par M.C. MARTINI et al.) par les hydrolysats I, II et III.

Le principe de la technique consiste en la mesure polarographique des pressions d'oxygène dans le milieu. En présence de cellules vivantes, donc consommant de l'oxygène, la pression en oxygène du milieu diminue et cette diminution est enregistrée en fonction du temps.

L'appareil utilisé est un analyseur d'oxygène (marque BECKMANN) équipé d'une électrode à oxygène de type CLARKE (BECKMANN 39 557). La cellule est constituée d'un tube de verre cylindrique, ayant 16 mm de diamètre interne et 43 mm de hauteur et est thermostatée à 38°C. L'extrémité extérieure est munie d'un rodage permettant un raccord étanche avec l'électrode.

Les cellules hépatiques sont obtenues par broyage du foie de souris 10PS OF1 femelles, non consanguines, de 7 semaines, dans du liquide de RINGER (1 ml pour 50 mg de foie). On introduit dans la cellule un volume de broyat permettant d'obtenir une consommation d'$O_2$ comprise entre 0,7 et 1,6 ppm en 10 mn (entre 1,5 et 2,5 ml de broyat selon le foie).

Les deux valeurs de consommation d'oxygène, témoin et essai, sont déterminées sur la même prise d'essai du broyat hépatique. La consommation d'oxygène est enregistrée pendant 10 mn, ce qui correspond à la valeur témoin. Un volume convenable de produit à tester est ajouté sous agitation. L'enregistrement est poursuivi pendant 10 mn, ce qui correspond à la valeur d'essai.

Dans les mêmes conditions, on a testé un extrait placentaire (lots A à M) actuellement commercialisé en France et incorporé dans diverses spécialités cosmétiques.

Les résultats obtenus avec l'extrait placentaire et hydrolysats de globine sont rassemblés dans le tableau II.

Dans les conditions testées, à des concentrations en matières protéiques du même ordre,les hydrolysats I,II,III induisent une stimulation de la consommation d'oxygène des cellules hépatiques supérieure ou égale à l'effet induit par l'extrait placentaire.

4) Stimulation de l'incorporation de thymidine tritiée dans l'ADN de fibroblastes cutanés.

La culture de fibroblastes cutanés humains est réalisée à partir de biopsies effectuées sur des donneurs. Les fibroblastes cutanés sont maintenus en culture pendant plusieurs subcultures (4 à 10).

Au jour 0 (J0), 50000 cellules sont ensemencées par puits (plaques FALCON 24 puits) dans le milieu suivant :

HAM F 10 + pénicilline 150 U/ml + streptomycine 150 U/ml + fongizone 100 μg/ml + SVF 12 %.

A J7, on remplace le milieu par du HAM F 10 + 1 % SVF. A J9 on ajoute les échantillons à tester ainsi que 1 μCi/ml de thymidine. A J10, les puits sont lavés par de la solution saline de PUCK et les cellules sont récupérées par grattage à l'aide d'un coton-tige. Les cotons-tiges subissent successivement, à + 4°C, 1 bain de TCA 10% et 2 bains de TCA 5%, pendant 30 mn pour chaque bain; ils sont ensuite séchés 1 nuit à 60°C.

L'extrémité radioactive des cotons-tiges est coupée et plongée dans 2 ml de liquide scintillant pour comptage.

Les résultats obtenus avec l'hydrolysat de globine sont rassemblés dans le tableau III.

L'hydrolysat de globine est capable d'induire la stimulation de l'incorporation de la thymidine dans la majorité des souches de fibroblastes cutanés testées. Sur les mêmes souches et dans les mêmes conditions, il n'a pas été possible de mettre en évidence un effet similaire avec l'extrait placentaire.

Les hydrolysats de globine appliqués selon l'invention permettent également de préparer des produits pharmaceutiques à base d'hydrolysat ou constitués par lui, qui sont efficace en dermatologie, notamment pour la cicatrisation. De préférence, les hydrolysats de globines de ces produits pharmaceutiques ont été épurés des facteurs de croissance cellulaire du type cytokines, s'ils étaient présents.

TABLEAU I

| | Milieu Témoin | Hydrolysat de globine I | | | EXTRAIT PLACENTAIRE | |
|---|---|---|---|---|---|---|
| | | 0,5 mg/ml | 1 mg/ml | 6 mg/ml | 0,4 mg/ml | 4,8 mg/ml |
| Nombre de cellules par puits (CCL 39) | $(55 \pm 16)$ $10^3$ | $(71 \pm 7)$ $10^3$ | $(80 \pm 9)$ $10^3$ | $(290 \pm 22)\ 10^3$ | $(48 \pm 8)$ $10^3$ | $(72 \pm 10)$ $10^3$ |
| % stimulation de la prolifération cellulaire | - | + 29 % | + 45 % | + 427 % | - | + 31 % |
| Seuil de signification (test t) | - | NS* | 0,01 | < 0,001 | - | NS* |

(NS* : Non Significatif)

TABLEAU III

| TYPE DE CELLULES | Concentration en matières protéiques testée (mg/ml) (hydrolysat I) | Incorporation de Thymidine par rapport au témoin | Seuil signification (test t) |
|---|---|---|---|
| 6627 F01 10e P. (enfant 9 ans) | 0,43 | + 136 % | 0,05 |
| 6670 F 01 (enfant 4 mois) | 2,2 | + 59 % | 0,05 |
| 6804 F 01 (adulte) | 2,2 | + 15 % | non significatif |
| 6805 F 01 (adulte) | 2,2 | + 43 % | 0,02 |

TABLEAU II

| TYPE D'EXTRAIT | Concentration en matière protéique testée (mg/ml) | Consommation O2 par rapport au témoin | Seuil signification (test t) |
|---|---|---|---|
| EXTRAIT PLACENTAIRE lot A | 7,5 | + 45 % | < 0,001 |
| B | 7,5 | + 58 % | < 0,001 |
| C | 7,5 | + 37 % | 0,01 |
| D | 35,6 | + 109 % | < 0,001 |
| E | 34,4 | + 113 % | < 0,001 |
| F | 36,9 | + 63 % | < 0,001 |
| G | 28,1 | + 50 % | 0,01 |
| H | 7,5 | + 46 % | < 0,001 |
| I | 30 | + 41 % | < 0,001 |
| J | 30,6 | + 54 % | < 0,001 |
| K | 33,1 | + 45 % | < 0,001 |
| L | 33,1 | + 35 % | 0,01 |
| M | 7,5 | + 137 % | < 0,001 |
| I HYDROLYSAT GLOBINE autoclavé 40 mn à 120°C | 4,4 | + 126 % | < 0,001 |
| | 0,9 | + 36 % | 0,01 |
| III HYDROLYSAT GLOBINE Lot A autoclavé 3h30 à 120°C | 4 | + 117 % | <0,001 |
| | 0,8 | + 128 % | <0,001 |
| | 3,7 | + 66 % | <0,001 |
| Lot B | 0,7 | + 121 % | <0,001 |
| II HYDROLYSAT GLOBINE autoclavé 1h à 132°C | 4,2 | + 124 % | <0,001 |
| | 0,8 | + 112 % | <0,001 |

**Revendications**

1. Application d'hydrolysats de globines, tels que, notamment, des extraits de cruor, à la préparation de produits pharmaceutiques, notamment pour la cicatrisation, et à la préparation de produits cosmétiques d'activation du métabolisme et de la multiplication cellulaires pour les soins des cheveux et/ou de la peau.

2. Application selon la revendication 1, caractérisée en ce que l'on utilise les hydrolysats répondant positivement à un test dans lequel on met la substance ou préparation de globine hydrolysée ou provenant de l'hydrolysat en présence de cellules animales contenues dans un milieu de culture dépourvu de sérum animal, on laisse incuber, puis on contrôle l'existence et, éventuellement, l'ampleur d'une multiplication desdites cellules par rapport à un milieu témoin ne recevant pas la substance ou la préparation testée.

3. Procédé de test de l'activité d'hydrolysats de globines, tels que, notamment, des extraits de cruor, caractérisé en ce qu'on met l'hydrolysat en présence de cellules animales contenues dans un milieu de culture dépourvu de sérum animal, on laisse incuber, puis on contrôle l'existence et, éventuellement, l'ampleur d'une multiplication desdites cellules par rapport a un milieu témoin ne recevant pas l'hydrolysat testé.

4. Procédé de test selon la revendication 3, caractérisé en ce que l'on élimine au préalable, s'ils sont présents dans l'hydrolysat, les facteurs de croissance cellulaire du type cytokines, et notamment les facteurs tels que FGF, PDGF, EGF.

5. Procédé de test selon l'une des revendications 3 et 4, caractérisé en ce que les cellules animales utilisées sont des fibroblastes.

6. Procédé de test selon la revendication 5, caractérisé en ce qu'il s'agit de fibroblastes cultivés dans un milieu de culture comprenant un mélange DMEM/HAM F12 additionné de 5 $\mu$g/ml d'insuline, de 5 $\mu$g/ml de transferrine, de $3.10^{-8}$M de sélénite, de 2 mg/ml d'albumine et de 2 $\mu$g/ml de fibronectine et de 50 $\mu$g/ml de gentamycine.

7. Procédé de test selon la revendication 6, caractérisé en ce qu'on incube la culture avec l'hydrolysat à raison de 50 000 cellules environ par ml de milieu, pendant environ 5 jours à 37°C, en ambiance humide et en présence de 5% environ de $CO_2$, puis on effectue un comptage des cellules.

8. Procédé de test selon la revendication 7, caractérisé en ce qu'on change le milieu de culture au deuxième jour de l'incubation.

9. Procédé de test selon l'une quelconque des revendications 5 à 8, caractérisé en ce que les fibroblastes sont des fibroblastes diploïdes de hamster.

10. Procédé de test selon l'une quelconque des revendications 3 à 9, caractérisé en ce qu'on teste en outre la stimulation de l'incorporation de thymidine tritiée dans l'ADN de cellules animales.

11. Procédé de test selon la revendication 10, caractérisé en ce que les cellules utilisées dans le test sont des fibroblastes.

12. Procédé de test selon la revendication 11, caractérisé en ce que les fibroblastes sont des fibroblastes cutanés humains.

13. Procédé de test selon la revendication 11 ou 12, caractérisé en ce que les fibroblastes sont ensemencés dans un milieu de culture comprenant du HAM F10 additionné de 150 U/ml de pénicilline, de 150 U/ml de streptomycine, de 100 $\mu$g/ml de fongizone et de 12% de sérum de veau foetal SVF.

14. Procédé de test selon la revendication 13, caractérisé en ce que, au jour 0, on ensemence environ 50 000 cellules par ml de milieu de culture ; au jour 7, on remplace le milieu par du HAM F10 additionné de 1% de SVF ; au jour 9, on ajoute l'hydrolysat à tester ainsi que 1 $\mu$Ci/ml de thymidine tritiée ; au jour 10, on effectue un lavage puis on mesure la radioactivité.

15. Procédé selon l'une quelconque des revendications 3 à 14, caractérisé en ce qu'en cas de présence de facteurs de croissance cellulaires, ceux-ci sont éliminés par hydrolyse enzymatique suivie d'un chauffage, notamment à 120°C pendant 30 à 40 mn.

16. Application à titre d'agents à usage externe en dermatologie et/ou en cosmétologie d'hydrolysats selon la revendication 1 ou 2, caractérisée en ce qu'ils répondent positivement au test selon l'une des revendications 3 à 15.

17. Application selon la revendication 16, caractérisée en ce qu'il s'agit d'extraits de cruor.

18. Produits cosmétiques d'activation du métabolisme et de la multiplication cellulaires pour les soins des cheveux et/ou de la peau, caractérisés en ce qu'ils comportent une ou plusieurs substances d'hydrolysats de globine ou des produits en dérivant.

19. Produits pharmaceutiques de dermatologie, notamment pour la cicatrisation, caractérisés en ce qu'ils comportent une ou plusieurs substances d'hydrolysats de globine ou des produits en dérivant.

20. Produits selon la revendication 18 ou 19, caractérisés en ce qu'ils sont capables de répondre positivement au procédé de test selon l'une quelconque des revendications 3 à 15.

21. Produits selon l'une quelconque des revendications 18 à 20, caractérisés en ce que ces substances sont des extraits de cruor.

22. Produits selon l'une quelconque des revendications 18 à 21, caractérisés en ce qu'ils sont dépourvus de facteurs de croissance cellulaire de type cytokines.

8

23. Procédé de préparation d'hydrolysats de globine tels que, notamment, d'extraits de cruor, caractérisé en ce qu'on chauffe la préparation d'hydrolysat de façon que celle-ci réponde positivement ou négativement au test selon l'une quelconque des revendications 3 à 9.

Office européen
des brevets

**RAPPORT DE RECHERCHE EUROPEENNE**

Numero de la demande

EP 90 40 2447

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | EP-A-0 237 398 (MOET-HENNESSY)<br>* Page 3, ligne 30 - page 4, ligne 14;<br>page 12, ligne 26 - page 17, ligne 12 *<br>--- | 1-23 | A 61 K 37/18<br>A 61 K 35/14<br>C 12 Q 1/02 |
| Y | FR-A-2 628 118 (CNRS ET UNIVERSITE DES SCIENCES ET TECHNIQUES LILLE I)<br>* En entier *<br>--- | 1-23 | |
| A | EP-A-0 061 556 (AB PRIPPS BRYGGERIER)<br>----- | | |

**DOMAINES TECHNIQUES RECHERCHES (Int. Cl.5)**

A 61 K

Le présent rapport a été établi pour toutes les revendications

| Lieu de la recherche | Date d'achèvement de la recherche | Examinateur |
|---|---|---|
| LA HAYE | 10-10-1990 | TURMO Y BLANCO C.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

.......................................................................
& : membre de la même famille, document correspondant

EPO FORM 1503 03.82 (P0402)